(19)

**Europäisches Patentamt**
**European Patent Office**
**Office européen des brevets**

(11) **EP 4 437 954 A1**

(12) **EUROPEAN PATENT APPLICATION**
published in accordance with Art. 153(4) EPC

(43) Date of publication:
**02.10.2024 Bulletin 2024/40**

(51) International Patent Classification (IPC):
***A61B 5/1455*** (2006.01)

(21) Application number: **22894215.7**

(86) International application number:
**PCT/CN2022/097242**

(22) Date of filing: **07.06.2022**

(87) International publication number:
**WO 2023/087672 (25.05.2023 Gazette 2023/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.11.2021 CN 202111386652**

(71) Applicant: **Lepu Medical Technology (Beijing) Co., Ltd.**
**Beijing 102200 (CN)**

(72) Inventors:
• **CAO, Jun**
  **Beijing 102200 (CN)**
• **ZHANG, Biying**
  **Beijing 102200 (CN)**
• **WANG, Sihan**
  **Beijing 102200 (CN)**

(74) Representative: **Glück Kritzenberger**
**Patentanwälte PartGmbB**
**Franz-Mayer-Str. 16a**
**93053 Regensburg (DE)**

(54) **BLOOD GLUCOSE PREDICTION METHOD AND DEVICE BASED ON OPTICAL SIGNAL FEATURES AND METABOLIC THERMAL FEATURES**

(57)     A blood glucose prediction method and device based on optical signal features and metabolic heat features. The method comprises: acquiring first calibrated blood glucose data (1); acquiring and generating first, second, third and fourth optical signals (2); acquiring and generating first, second, third, fourth, fifth, sixth and seventh metabolic heat signals (3); extracting and normalizing human body optical signal features to generate first, second and third optical feature data groups (4); extracting and normalizing environmental optical signal features to generate fourth optical feature data (5); extracting and normalizing metabolic heat features to generate first, second, third, fourth, fifth, sixth and seventh metabolic heat feature data (6); performing feature fusion to generate a first feature vector (7); predicting, on the basis of a floating blood glucose prediction model, floating blood glucose values to generate first floating blood glucose data (8); and generating first predicted blood glucose data according to the sum of the first calibrated blood glucose data and the first floating blood glucose data (9). The blood glucose prediction method and device based on optical signal features and metabolic heat features can be used to provide a non-invasive blood glucose measurement mechanism.

FIG. 1

**Description**

CROSS-REFERENCE TO RELATED APPLICATION

This application Claims priority to Chinese Patent Application No. 202111386652.X, filed to the China Patent Office on November 22, 2021 and entitled "BLOOD GLUCOSE PREDICTION METHOD AND DEVICE BASED ON OPTICAL SIGNAL FEATURES AND METABOLIC HEAT FEATURES".

BACKGROUND OF THE INVENTION

1. Technical Field

The present invention relates to the technical field of data processing, and in particular to a blood glucose prediction method and device based on optical signal features and metabolic heat features.

2. Description of Related Art

Glucose in blood is known as blood glucose (Glu). The blood glucose should be kept at certain level to maintain the normal work of various organs and tissues in a body. Short-term instability of the blood glucose may lead to discomfort in the organs and tissues to different extents, and long-term instability may lead to lesions in the organs and tissues to different extents. Therefore, a measured blood glucose value is a very important parameter for health evaluation in the field of health surveillance. At present, the common means for blood glucose measurement is mainly based on an invasive method, by which the blood is collected from a human body and then chemically analyzed to obtain a measured blood glucose value. Such a means for measurement causes a wound to the human body. Therefore, if the blood glucose in a human body is measured for a long term by this means, it will undoubtedly bring a lot of inconvenience to users, for example, wound pain, wound infection, or other adverse experience.

Based on the Beer-Lambert law, it can be known that the absorbency of a solution for transmitted light is related to the solute concentration, and thus the higher the concentration of glucose in blood, the smaller the intensity of light transmitting through the tissues in a human body. Then, the changes of blood glucose can be measured by acquiring photoplethysmography (PPG) signals reflecting the changes in the intensity of light transmitting through the blood in the human body.

Based on the metabolic heat conformation (MHC) theory, it can be known that the energy rhythms in a human body in different time periods show certain correlation with the energy released after the metabolism of the human body, and the oxidized glucose and the produced energy can be dissipated from the human body to the environment in the form of heat energy. Accordingly, the metabolic heat in the human body is related to the glucose level and oxygen supply. That is, with a secured oxygen supply, the blood glucose changes can be measured based on the MHC theory, with the human metabolic heat as a reference.

BRIEF SUMMARY OF THE INVENTION

In view of the defects in the prior art, an object of the present invention is to provide a blood glucose prediction method and device based on optical signal features and metabolic heat features, an electronic apparatus, and a computer-readable storage medium. With the present invention, a non-invasive measurement mechanism can be provided for long-term blood glucose measurement, which reduces inconvenience brought to users and decreases the difficulty in measurement, thereby improving user experience.

To achieve the above object, a first aspect of embodiments of the invention provides a blood glucose prediction method based on optical signal features and metabolic heat features. The method includes:

acquiring a calibrated blood glucose value to generate first calibrated blood glucose data;
according to a preset optical signal acquisition duration, a human body optical signal type and three human body optical signal bands, continuously acquiring human body optical signals to generate corresponding first, second, and third optical signals, and according to the optical signal acquisition duration, continuously acquiring environmental optical signals to generate a fourth optical signal;
according to a preset metabolic heat signal acquisition duration, continuously acquiring human body contact heat signals to generate a first metabolic heat signal, continuously acquiring radiation heat signals from a proximal end of a human body to generate a second metabolic heat signal, continuously acquiring temperature change information from the proximal end of the human body to form a third metabolic heat signal, continuously acquiring humidity change information from the proximal end of the human body to form a fourth metabolic heat signal, continuously acquiring calibration output information from a proximal-end radiation sensor used to acquire the radiation heat signals to form a fifth metabolic heat signal, continuously acquiring temperature change information from a distal end of the human body to form a sixth metabolic heat signal, and continuously acquiring humidity change information from the distal end of the human body to form a seventh metabolic heat signal;
extracting and normalizing human body optical signal features of the first, second, and third optical signals to generate corresponding first, second, and third optical feature data groups;
extracting and normalizing environmental optical signal features of the fourth optical signal to generate corresponding fourth optical feature data;
extracting and normalizing metabolic heat features of the first, second, third, fourth, fifth, sixth, and seventh metabolic heat signals to generate corresponding first, second, third, fourth, fifth, sixth, and seventh metabolic heat feature data;
performing feature fusion on all the obtained feature data to generate a first feature vector; and
based on a floating blood glucose prediction model, predicting floating blood glucose values for the first feature vector to generate first floating blood glucose data; and generating first predicted blood glucose data according to a sum of the first calibrated blood glucose data and the first floating blood glucose data.

Preferably, the optical signal type includes a photoplethysmography signal type; and
the three optical signal bands include an infrared wave band of 650 nm, a near-infrared wave band of 940 nm, and a near-infrared wave band of 1050 nm.

Preferably, extracting and normalizing the human body optical signal features of the first, second, and third optical signals to generate the corresponding first, second, and third optical feature data groups specifically includes:

extracting a first specified duration of optical signal segment from the first, second, or third optical signal in an intermediate signal time period to generate a first segment signal;
according to a preset bandpass filtering frequency band, performing bandpass filtering on the first segment signal to generate a corresponding first filtered signal, recognizing peak values of the first filtered signal to obtain a plurality of corresponding first signal peak value data, averaging the plurality of first signal peak value data to generate corresponding first averaged peak value data, flipping the first filtered signal upside down to generate a corresponding first flipped signal, recognizing peak values of the first flipped signal to obtain a plurality of corresponding second signal peak value data, averaging the plurality of second signal peak value data and inverting an averaged result to generate corresponding first averaged valley value data, subtracting the first averaged valley value data from the first averaged peak value data to generate corresponding first feature data, and normalizing the first feature data to generate alternating-current feature data;
according to a preset low-pass filtering frequency threshold, performing low-pass filtering on the first segment signal to generate a corresponding second filtered signal, averaging the second filtered signal to generate corresponding second feature data, and normalizing the second feature data to generate direct-current feature data; and
forming the corresponding first, second, or third optical feature data groups from the obtained alternating-current feature data and direct-current feature data.

Preferably, extracting and normalizing the environmental optical signal features of the fourth optical signal to generate the corresponding fourth optical feature data specifically includes:

extracting a first specified duration of optical signal segment from the fourth optical signal in an intermediate signal time period to generate a second segment signal;
averaging the second segment signal to generate third feature data; and
normalizing the third feature data to generate the fourth optical feature data.

Preferably, extracting and normalizing the metabolic heat features of the first, second, third, fourth, fifth, sixth, and seventh metabolic heat signals to generate the corresponding first, second, third, fourth, fifth, sixth, and seventh metabolic heat feature data specifically includes:

extracting a second specified duration of metabolic heat signal segment from the first, second, third, fourth, fifth, sixth, or seventh metabolic heat signal in a last signal time period to generate a corresponding third segment signal;
averaging the third segment signal to generate corresponding fourth feature data; and
normalizing the fourth feature data to generate the corresponding first, second, third, fourth, fifth, sixth, or seventh metabolic heat feature data.

Preferably, the floating blood glucose prediction model is implemented based on a network structure of a multilayer neural network, and includes an input layer, a hidden layer, and an output layer, wherein the input layer includes a first number M of input nodes, the hidden layer includes a second number N of hidden-layer input nodes and a third number S of hidden-layer output nodes, the output layer includes an output node, the individual hidden-layer input nodes of the hidden layer are connected to all the input nodes of the input layer, respectively, to form a corresponding first fully connected network, the individual hidden-layer output nodes of the hidden layer are connected to all the hidden-layer input nodes of the hidden layer, respectively, to form a corresponding second fully connected network, the output node of the output layer is connected to all the hidden-layer input nodes, the first number M is associated with a sum of feature classifications of the first feature vector, and the second number N is greater than the third number S which is greater than 0;

during prediction of the floating blood glucose values by the floating blood glucose prediction model, the input layer is called to extract individual feature data of the first feature vector in sequence and then input to the corresponding input nodes to generate corresponding input node data;

the hidden layer is called to perform, based on individual parameter matrices of the first fully connected network, corresponding first fully connected network computation on all the input node data connected to the first fully connected network, and to input computation results to the corresponding hidden-layer input nodes as hidden-layer input node data;

the hidden layer is called to perform, based on individual parameter matrices of the second fully connected network, corresponding second fully connected network computation on all the hidden-layer input node data connected to the second fully connected network, and to input computation results to the corresponding hidden-layer output nodes as hidden-layer output node data; and

the output layer is called to perform, based on a preset activation function, corresponding activation function computation on all the hidden-layer output node data and to output computation results as the first floating blood glucose data.

A second aspect of the embodiments of the invention provides a device for implementing steps of the blood glucose prediction method based on optical signal features and metabolic heat features as defined above in the first aspect. The device includes: an acquisition module, a first data acquisition module, a second data acquisition module, a first feature data processing module, a second feature data processing module, a feature data fusion module, and a blood glucose prediction module;

the acquisition module is configured to acquire a calibrated blood glucose value to generate first calibrated blood glucose data;

the first data acquisition module is configured to, according to a preset optical signal acquisition duration, a human body optical signal type and three human body optical signal bands, continuously acquire human body optical signals to generate corresponding first, second, and third optical signals, and according to the optical signal acquisition duration, continuously acquire environmental optical signals to generate a fourth optical signal;

the second data acquisition module is configured to, according to the preset metabolic heat signal acquisition duration, continuously acquire human body contact heat signals to generate a first metabolic heat signal, continuously acquire radiation heat signals from a proximal end of a human body to generate a second metabolic heat signal, continuously acquire temperature change information from the proximal end of the human body to form a third metabolic heat signal, continuously acquire humidity change information from the proximal end of the human body to form a fourth metabolic heat signal, continuously acquire calibration output information from a proximal-end radiation sensor used to acquire the radiation heat signals to form a fifth metabolic heat signal, continuously acquire temperature change information from a distal end of the human body to form a sixth metabolic heat signal, and continuously acquire humidity change information from the distal end of the human body to form a seventh metabolic heat signal;

the first feature data processing module is configured to extract and normalize human body optical signal features of the first, second, and third optical signals to generate corresponding first, second, and third optical feature data groups, and extract and normalize environmental optical signal features of the fourth optical signal to generate corresponding fourth optical feature data;

the second feature data processing module is configured to extract and normalize metabolic heat features of the first, second, third, fourth, fifth, sixth, and seventh metabolic heat signals to generate corresponding first, second, third, fourth, fifth, sixth, and seventh metabolic heat feature data;

the feature data fusion module is configured to perform feature fusion on all the obtained feature data to generate a first feature vector; and

the blood glucose prediction module is configured to, based on a floating blood glucose prediction model, predict floating blood glucose values for the first feature vector to generate first floating blood glucose data, and generate first predicted blood glucose data according to a sum of the first calibrated blood glucose data and the first floating blood glucose data.

A third aspect of the embodiments of the invention provides an electronic apparatus, including: a memory, a processor, and a transceiver, wherein

the processor is configured to be coupled with the memory to read and execute an instruction in the memory for implementing the steps of the method as defined above in the first aspect;

the transceiver is coupled with the processor, which controls the transceiver to transceive a message.

A fourth aspect of the embodiments of the invention provides a computer-readable storage medium storing a computer instruction, wherein the computer instruction, when executed by a computer, enables the computer to execute the instruction for the method as defined above in the first aspect.

The embodiments of the invention provide a blood glucose prediction method and device based on optical signal features and metabolic heat features, an electronic apparatus, and a computer-readable storage medium, whereby the human body photoplethysmography signals and environmental optical signals are acquired, the contact heat, radiation heat, temperature, humidity, and radiation sensor calibration signals related to the metabolic heat of the human body are acquired, and the ambient temperature and humidity signals related to the metabolic heat of the human body are acquired; furthermore, the features of the acquired optical and metabolic heat-related signals are extracted and normalized to form a feature vector; and the floating blood glucose prediction model reflecting the correlation among the optical, metabolic heat and blood glucose changes is used to perform prediction for the feature vector to obtain the corresponding floating blood glucose data, such that the predicted blood glucose data can be obtained based on the pre-acquired calibrated blood glucose values plus the floating blood glucose data. The invention provides, for example, a non-invasive measurement mechanism for long-term blood glucose measurement, which reduces for example inconvenience brought to users and decreases for example the difficulty in measurement, thereby improving the user experience.

## BRIEF DESCRIPTION OF THE SEVERAL VIEWS OF THE DRAWINGS

FIG. 1 is a schematic diagram of a blood glucose prediction method based on optical signal features and metabolic heat features according to a first embodiment of the present invention;
FIG. 2 is a schematic structural diagram of a neural network of a floating blood glucose prediction model according to the first embodiment of the present invention;
FIG. 3 is a modular structural diagram of a blood glucose prediction device based on optical signal features and metabolic heat features according to a second embodiment of the present invention; and
FIG. 4 is a schematic structural diagram of an electronic apparatus according to a third embodiment of the present invention.

## DETAILED DESCRIPTION OF THE INVENTION

For the clearer description of the objects, technical solutions and advantages of the invention, the following further describes the technical solutions of the present disclosure in detail in combination with the accompanying drawings. Apparently, some instead of all of the embodiments of the invention are merely described. Based on the embodiments of the invention, every other embodiment that can be achieved by a person of ordinary skills in the art without creative efforts shall fall within the protection scope of the invention.

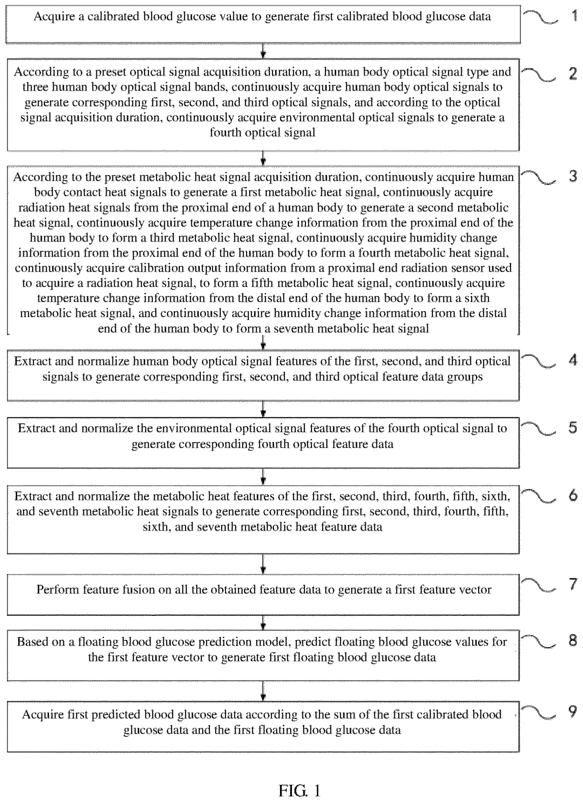

The first embodiment of the present invention provides a blood glucose prediction method based on optical signal features and metabolic heat features, as shown in FIG. 1 which is a schematic diagram of the blood glucose prediction method based on optical signal features and metabolic heat features according to the first embodiment of the invention. The method mainly includes the following steps.

In Step 1, a calibrated blood glucose value is acquired to generate first calibrated blood glucose data.

It should be noted that, before the current step, the method in the embodiment of the present invention includes calibrating blood glucose in advance to generate the calibrated blood glucose value, which includes the following steps.

In Step A1, one or more measured blood glucose data are obtained by conventional blood glucose detection, which is performed on a current user one or more times by a conventional means for blood glucose detection; the time information of current blood glucose detection and the basic user physiological information are recorded, and related optics signals and metabolic heat signals are acquired and recorded during the current blood glucose detection; and corresponding calibrated reference data groups are formed from the measured blood glucose data, time information, basic user physiological information, related optics- signals, and metabolic heat related signals from each detection.

The basic user physiological information includes age, gender, height, body weight, body mass index (BMI) or the like; the optics related signals include human body photoplethysmography signals and environmental optical signals in three optical signal wave bands (an infrared wave band of 650 nm, a near-infrared wave band of 940 nm, and a near-infrared wave band of 1050 nm); and the metabolic heat related signals include human body contact heat signals, radiation heat signals at the proximal end of a human body, temperature change signals at the proximal end of the human body, humidity change signals at the proximal end of the human body, calibration output signals of a proximal-end radiation sensor used to acquire radiation heat signals, temperature change signals at a distal end of the human body, and humidity change signals at the distal end of the human body.

In Step A2, in case of only one conventional blood glucose detection that has been performed on the user, the measured blood glucose data arising from the current detection are taken as a calibrated blood glucose value and stored, and the data, other than the measured blood glucose data, in the corresponding calibration reference data groups are taken as a calibration condition data group and stored.

In Step A3, in case of multiple conventional blood glucose detections that have been performed on the user, a plurality of obtained measured blood glucose data is averaged to generate corresponding averaged measured blood glucose data; the measured blood glucose data having the minimal difference from the averaged measured blood glucose data are taken as the calibrated blood glucose value and stored, and the data, other than the measured blood glucose data, in the calibration reference data groups corresponding to the calibrated blood glucose value are taken as a calibration condition data group and stored.

Here, once created, the calibrated blood glucose value and the calibration condition data group are saved as benchmark data for a current user. During each subsequent blood glucose measurement to be performed on the current user based on the embodiment of the invention, the blood glucose fluctuations of the current user will be predicted by taking the calibration condition data group of the benchmark data as a reference for the prediction model, to obtain the corresponding floating blood glucose data; and the predicted blood glucose data of the current user may be obtained from the calibrated blood glucose value of the benchmark data plus the floating blood glucose data. That is, after the benchmark data of the current user are created, the process of each subsequent blood glucose measurement to be performed on this user is a non-invasive measurement and processing process.

In Step 2, according to a preset optical signal acquisition duration, a human body optical signal type and three human body optical signal bands, human body optical signals are continuously acquired to generate corresponding first, second, and third optical signals; and according to the optical signal acquisition duration, environmental optical signals are continuously acquired to generate a fourth optical signal.

Here, the optical signal type includes a photoplethysmography signal type; and the three optical signal wave bands include an infrared wave band of 650 nm, a near-infrared wave band of 940 nm, and a near-infrared wave band of 1050 nm.

Here, to guarantee the acquisition quality for the optical signals, the duration for acquiring the optical signals is generally set to 30 sec or 1 min, or may be separately set based on the acquisition quality of a signal acquisition apparatus. During acquisition of the human body optical signals, to prevent different wave bands of light sources from interfering with each other, light-emitting diode (LED) lights are pressed close to the finger pulps of a human body for irradiation in sequence, and three corresponding photosensitive diodes are used at positions on the finger dorsum perpendicularly corresponding to the individual LED lights to sequentially acquire three wave bands of transmitted light signals, which are generally acquired in a chronological sequence according to a preset sampling frequency (generally, 125 Hz) by using three LEDs with the wave bands of the infrared wave band of 650 nm, the near-infrared wave band of 940 nm, and the near-infrared wave band of 1050 nm, respectively, thereby obtaining a first optical signal that is specifically the transmitted light of the infrared wave band of 650 nm, a second optical signal that is specifically the transmitted light of the infrared wave band of 940 nm, and the third optical signal that is specifically the transmitted light of the infrared wave band of 1050 nm. During acquisition of the environmental optical signals, to prevent interferences from the above LED light sources, the natural light signals of environmental background light are generally continuously acquired according to the preset sampling frequency (generally, 125 Hz) after the acquisition of the above first, second, and third optical signals is ended, thereby obtaining the fourth optical signal. The above first, second, third, and fourth optical signals are acquired at the same frequency, with the same duration.

In Step 3, according to a preset metabolic heat signal acquisition duration, human body contact heat signals are continuously acquired to generate a first metabolic heat signal; radiation heat signals are continuously acquired from a proximal end of a human body to generate a second metabolic heat signal; temperature change information is continuously acquired from the proximal end of the human body to form a third metabolic heat signal; humidity change information is continuously acquired from the proximal end of the human body to form a fourth metabolic heat signal; calibrated output information is continuously acquired from a proximal-end radiation sensor used to acquire the radiation heat signals to form a fifth metabolic heat signal; temperature change information is continuously acquired from a distal end of the human body to form a sixth metabolic heat signal; and humidity change information is continuously acquired from the distal end of the human body to form a seventh metabolic heat signal.

Here, to guarantee the synchronism between the metabolic heat signals and the optical signals, the metabolic heat signals are acquired in synchronization with the process of acquiring any of the first, second, and third optical signals, and at the moment, the corresponding acquisition duration for the metabolic heat signals is the same as the acquisition duration of the optical signals. The metabolic heat signals may also be acquired in synchronization with the whole process of acquiring the first, second, and third optical signals, and at the moment, the corresponding acquisition duration for the metabolic heat signals is greater than or equal to three times the acquisition duration of the optical signals. To further guarantee the consistent data accuracy between the metabolic heat signals and the optical signals, the sampling frequency for all the metabolic heat signals may be set to be the same as the sampling frequency (generally, 125 Hz) for the optical signals.

During continuous acquisition of the human body contact heat signals, a contact sensor is pressed close to the above finger pulp to acquire the heat signals.

During continuous acquisition of the radiation heat signals, temperature change information, and humidity change information at the proximal end of the human body, these signals are acquired using a proximal-end radiation sensor, a temperature sensor, and a humidity sensor, which are apart from the above finger pulp by certain tiny distance.

The calibration output information of the proximal-end radiation sensor is acquired for the purpose of providing a calibration signal for the acquired proximal-end radiation heat signals.

During continuous acquisition of the temperature change information and humidity change information at the distal end of the human body, these signals are acquired using a temperature sensor and a humidity sensor, which are apart from the above finger by a distance that is greater than the foregoing tiny distance to prevent the acquired ambient temperature and humidity from being affected by the temperature and humidity parameters of the human body.

In Step 4, human body optical signal features of the first, second, and third optical signals are extracted and normalized to generate corresponding first, second, and third optical feature data groups.

The first, second, and third optical feature data groups each consist of alternating-current feature data and direct-current feature data.

Here, extracting the human body optical signal features is in effect to extract the alternating- and direct-current features of three human body photoplethysmography signals.

Specifically, the following steps are included. In Step 41, a first specified duration of optical signal segment is extracted from the first, second, or third optical signal in an intermediate signal time period to generate a first segment signal.

Here, the first specified duration is generally 20 sec.

In Step 42, according to a preset bandpass filtering frequency band, bandpass filtering is performed on the first segment signal to generate a corresponding first filtered signal; peak values of the first filtered signal are recognized to obtain a plurality of corresponding first signal peak value data; the plurality of first signal peak value data is averaged to generate corresponding first averaged peak value data; the first filtered signal is flipped upside down to generate a corresponding first flipped signal; peak values of the first flipped signal are recognized to obtain a plurality of corresponding second signal peak value data; the plurality of second signal peak value data is averaged and an averaged result is inverted to generate corresponding first averaged valley value data; the first averaged valley value data is subtracted from the first averaged peak value data to generate corresponding first feature data; and the first feature data are normalized to generate alternating-current feature data.

Here, it is in effect to extract the alternating-current feature information of the first, second, and third optical signals. The bandpass filtering frequency band is generally of 0.5-10 HZ. The method of the embodiment of the present invention regards the signals of less than 0.5 Hz as direct-current feature signals, and the signals of higher than 10 Hz as interference or noise signals, and the alternating-current feature signals can be obtained by filtering and removing the direct-current feature signals and the interference or noise signals. The alternating-current feature signals are featured with a waveform amplitude difference, i.e., a peak-valley value difference, and the alternating-current feature data computed here are actually the average amplitude data of the alternating-current feature signals.

Further, during normalization of the first feature data to generate the alternating-current feature data, the embodiment of the present invention provides at least two types of normalizing flow processes according to a preset normalization mode on the basis of a large number of existing training data used for training a subsequent prediction model. Specifically, the following steps are included.

In Step B1, when the normalization mode is a first mode, the first feature data are normalized by using an extreme-value normalization function.

The extreme-value normalization function is

$$X_{norm} = \frac{X - X_{min}}{X_{max} - X_{min}}$$

, with $X_{norm}$ representing normalized data, X representing inputted first feature data, and $X_{min}$ and $X_{max}$ representing minimum and maximum values of the training data that have the same type as the first feature data and are in the data group consisting of a large number of above training data, respectively.

In Step B2, when the normalization mode is a second mode, the first feature data are normalized by using the mean variance normalization function.

The mean variance normalization function is

$$X_{norm} = \frac{X - \mu}{\sigma}$$

, with $X_{norm}$ representing normalized data, X representing inputted first feature data, and $\mu$ and $\sigma$ representing the average value and standard deviation of the training data that have the same type as the first feature data and are in the data group consisting of a large number of above training data, respectively.

In Step 43, according to a preset low-pass filtering frequency threshold, low-pass filtering is performed on the first segment signal to generate a corresponding second filtered signal; the second filtered signal is averaged to generate corresponding second feature data; and the second feature data is normalized to generate direct-current feature data.

Here, it is in effect to extract the direct-current feature information from the first, second, and third optical signals. The low-pass filtering frequency threshold is generally 0.5 Hz. The direct-current feature signals are obtained by retaining the signals of less than 0.5 Hz, and the alternating-current feature signals are featured in that a waveform is substantially flat without obvious peak-valley changes. The direct-current feature data calculated here are the averaged amplitude data of the direct-current feature signals.

During normalization of the second feature data to generate the direct-current feature data, its normalization process is similar to that of the first feature data in Step 41, the details of which will not be further repeated.

In Step 44, the corresponding first, second, or third optical feature data groups are formed from the obtained alternating-current feature data and direct-current feature data.

In Step 5, environmental optical signal features of the fourth optical signal are extracted and normalized to generate corresponding fourth optical feature data.

Wherein it includes: extracting a first specified duration of optical signal segment from the fourth optical signal in an intermediate signal time period to generate a second segment signal; averaging the second segment signal to generate third feature data; and normalizing the third feature data to generate fourth optical feature data.

Here, the third feature data may reflect the average intensity of the ambient background light; and the process of normalizing the third feature data to generate the fourth optical feature data is similar to the process of normalizing the first feature data in Step 41, the details of which will not be repeated here.

In Step 6, metabolic heat features of the first, second, third, fourth, fifth, sixth, and seventh metabolic heat signals are extracted and normalized to generate corresponding first, second, third, fourth, fifth, sixth, and seventh metabolic heat feature data.

Wherein, the following steps are included. In Step 61, a second specified duration of metabolic heat signal segment is extracted from the first, second, third, fourth, fifth, sixth, or seventh metabolic heat signal in a last signal time period to generate a corresponding third segment signal.

Here, since these metabolic heat-related signals are mostly slowly changing signals, and the corresponding sensor acquired data present a slowly changing trend, certain time may be required to reach a stable value again, and the fragment of the metabolic heat signals are obtained by truncating the finally specified length of fragment; and the second specified duration is generally 10 sec.

In Step 62, the third segment signal is averaged to generate corresponding fourth feature data.

Here, if the third segment signal is the segment data of the first metabolic heat signals, the fourth feature data are an averaged value for human body contact heat; if the third segment signal is the segment data of the second metabolic heat signals, the fourth feature data are an averaged value for human body proximal-end radiation heat; if the third segment signal is the segment data of the third metabolic heat signals, the fourth feature data are an averaged value for human body proximal-end temperature; if the third segment signal is the segment data of the fourth metabolic heat signals, the fourth feature data are an averaged value for human body proximal-end humidity; if the third segment signal is the segment data of the fifth metabolic heat signals, the fourth feature data are an averaged calibration value of the proximal-end radiation sensor used to acquire the radiation heat signals; if the third segment signal is the segment data of the sixth metabolic heat signals, the fourth feature data are an averaged value for human body distal-end temperature, which is in effect the averaged ambient temperature value; and if the third segment signal is the segment data of the seventh metabolic heat signals, the fourth feature data are an averaged human body distal-end humidity value which is in effect an averaged ambient humidity value.

In Step 63, the fourth feature data are normalized to generate the corresponding first, second, third, fourth, fifth, sixth, or seventh metabolic heat feature data.

Here, during normalization of the fourth feature data, its normalization process is similar to that of the first feature data in Step 41, the details of which will not be further repeated. and in a case where the third segment signal is the segment data of the first, second, third, fourth, fifth, sixth, and seventh metabolic heat feature data respectively, the normalizing results are the corresponding first, second, third, fourth, fifth, sixth, and seventh metabolic heat feature data.

In Step 7, feature fusion is performed on all the obtained feature data to generate a first feature vector.

Here, according to the sequence requirement of the subsequent prediction model for the input data, 14 pieces of feature data, including the first, second and third optical feature data groups, the fourth optical feature data, as well as the first, second, third, fourth, fifth, sixth, and seventh metabolic heat feature data, are ranked, and the corresponding first feature vector is constructed according to a ranking result.

It should be noted that, in the particular case of the embodiment of the present invention, the current prediction time and the basic personal physiological information (including age, gender, height, body weight, and BMI) may also be acquired in real time, and the relevant acquired data can be incorporated into the first feature vector. The particular case includes taking the acquisition time as one of the blood glucose change factors, and the basic personal physiological information changes as one of the blood glucose change factors.

In Step 8, based on a floating blood glucose prediction model, floating blood glucose values are predicted for the first feature vector to generate first floating blood glucose data.

The floating blood glucose prediction model is implemented based on a network structure of a multilayer neural network, and includes an input layer, a hidden layer, and an output layer. The input layer includes a first number M of input nodes; the hidden layer includes a second number N of hidden-layer input nodes and a third number S of hidden-layer output nodes; the output layer includes an output node; the individual hidden-layer input nodes of the hidden layer are connected to all the input nodes of the input layer, respectively, to form a corresponding first fully connected network; the individual hidden-layer output nodes of the hidden layer are connected to all the hidden-layer input nodes of the hidden layer, respectively, to form a corresponding second fully connected network; the output node of the output layer is connected to all the hidden-layer input nodes; and the first number M is associated with a sum of feature classifications of the first feature vector, and the second number N is greater than the third number S which is greater than 0.

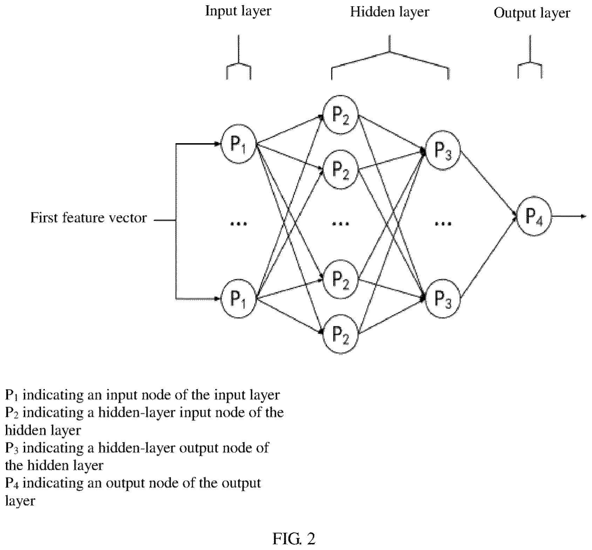

Here, the structure of the floating blood glucose prediction model in the embodiment of the invention is shown as in FIG. 2, which is a schematic structural diagram of a neural network of a floating blood glucose prediction model according to a first embodiment of the invention. Based on the Beer-Lambert law and the metabolic heat conformation theory, this model constructs the network computation model of the first and second fully connected networks and the structures of the parameter matrices required for computation; during each prediction of this prediction model, the number of input nodes of the input layer is set according to the data types of the first feature vector, a corresponding calibration feature vector is then constructed according to the corresponding relation between the calibration condition data group of the current user benchmark data and the data types of the first feature vector, and then, parameters are tuned for the parameter matrices of the first and second fully connected networks and for the preset activation function of the output layer according to the calibration feature vector; and then, the fluctuating blood glucose values of the current user at the current time are predicted according to the first feature vector to obtain the corresponding first floating blood glucose data.

It should be noted that, the data types of the first feature vector necessarily include the 14 pieces of feature data (including the first, second and third optical feature data groups, the fourth optical feature data, as well as the first, second, third, fourth, fifth, sixth, and seventh metabolic heat feature data,) produced from the above processing steps, and optionally, may also include the feature data such as time, age, gender, height, body weight, and BMI for the current prediction. During setting of the number of input nodes of the input layer according to the data types of the first feature vector, the corresponding input nodes needs to be increased or decreased according to the actual data types of the first feature vector.

It should be noted that when the corresponding calibration feature vector is constructed according to the corresponding relation between the calibration condition data group of the current user benchmark data and the data types of the first feature vector, the alternating- and direct-current feature data of the human body photoplethysmography signals in the three optical signal wave bands (i.e., the infrared wave band of 650 nm, the near-infrared wave band of 940 nm, and the near-infrared wave band of 1050 nm) in the calibration condition data group need to be extracted and then normalized in a way similar to that of Step 4 for extracting and normalizing the human body optical signal features of the first, second, and third optical signals, thereby obtaining 6 pieces of calibrated feature data of the human body optical signals; the feature data of the environmental optical signals in the calibration condition data group need to be extracted and normalized in a way similar to that of Step 5 for extracting and normalizing the environmental optical signal features of the fourth optical signal, thereby obtaining 1 piece of calibrated environmental optical feature data; the feature data of the human body contact heat signals, radiation heat signals at the proximal end of a human body, temperature change signals at the proximal end of the human body, humidity change signals at the proximal end of the human body, calibration output signals of the proximal-end radiation sensor used to acquire the radiation heat signals, temperature change signals at a distal end of the human body, and humidity change signals at the distal end of the human body need to be extracted and normalized in a way similar to that of Step 6 for extracting and normalizing the metabolic heat features of the first, second, third, fourth, fifth, sixth, and seventh metabolic heat signals, thereby obtaining 7 pieces of calibrated feature data of the metabolic heat signals; and in a case where the data types of the first feature vector further include the data type of features such as time, age, gender, height, body weight, or BMI, the corresponding calibrated feature data of the time, age, gender, height, body weight, or BMI are further generated according to the time information and basic user physiological information (including age, gender, height, body weight, and BMI) in the calibration condition data group, and then, are fused together with the foregoing 6 pieces of calibrated feature data of human body optical signals, 1 piece of calibrated environmental optical feature data, and 7 pieces of calibrated feature data of metabolic heat signals according to the same data ranking structure as the first feature vector, thereby obtaining the calibrated feature vector.

It should be noted that the floating blood glucose prediction model in the embodiment of the invention is formed by acquiring and training a large number of different individual data in advance, and thus, the prediction accuracy of the model is related to the population features to which the trained data belong. In a case where the prediction result of the model needs to be further related to personalized features, parameters need to be tuned for the parameter matrices of the first and second fully connected networks and for the preset activation function of the output layer according to the calibrated feature vector to achieve the purpose of personalized calibration, before the model is used; and after the personalized calibration is completed, the model may tune the prediction result according to the personalized benchmark features of a current individual, thereby bring the prediction result close to the real data of the current individual, which improves the personalized prediction accuracy of the model.

Further, predicting the floating blood glucose values by the floating blood glucose prediction model includes the following steps.

In Step C1, the input layer is called to extract individual feature data of the first feature vector in sequence and then input to the corresponding input nodes to generate corresponding input node data.

In Step C2, the hidden layer is called to perform, based on individual parameter matrices of the first fully connected network, corresponding first fully connected network computation on all the input node data connected to the first fully connected network, and to input computation results to the corresponding hidden-layer input nodes as hidden-layer input node data.

In Step C3, the hidden layer is called to perform, based on individual parameter matrices of the second fully connected network, corresponding second fully connected network computation on all the hidden-layer input node data connected to the second fully connected network, and to input computation results to the corresponding hidden-layer output nodes as hidden-layer output node data.

In Step C4, the output layer is called to perform, based on a preset activation function, corresponding activation function computation on all the hidden-layer output node data and to output computation results as the first floating blood glucose data.

In Step 9, first predicted blood glucose data are generated according to a sum of the first calibrated blood glucose data and the first floating blood glucose data.

Here, the predicted blood glucose value of the current user at the current moment, i.e., the first predicted blood glucose data, may be obtained by adding the calibrated blood glucose value based on the benchmark data of the current user, i.e., the first calibrated blood glucose data, to the floating blood glucose value output by the floating blood glucose prediction model, i.e., the first floating blood glucose data.

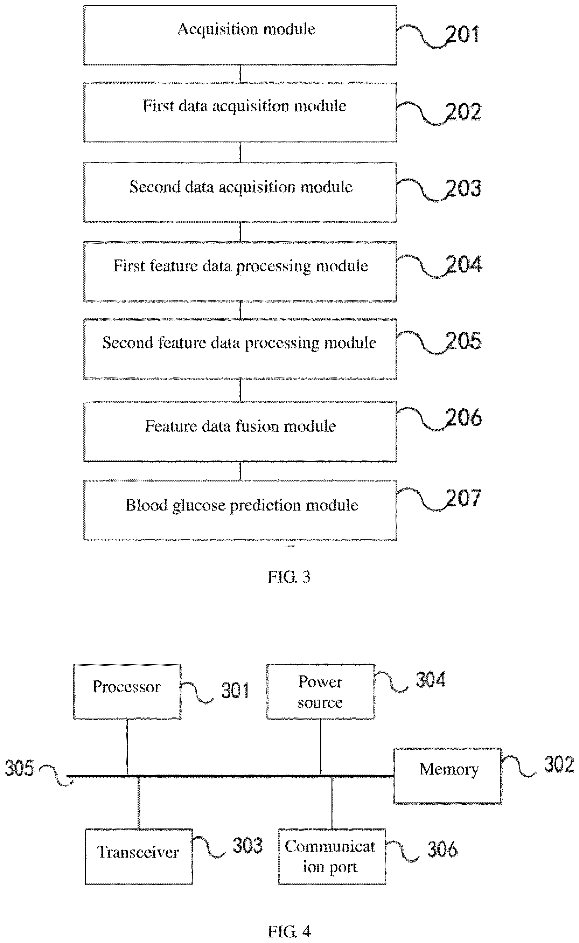

FIG. 3 is a modular structural diagram of a blood glucose prediction device based on optical signal features and metabolic heat features according to a second embodiment of the invention. The device may be a terminal unit or server for implementing the method according to the embodiment of the invention, or a device connected to the above-mentioned terminal unit or server for implementing the method according to the embodiment of the invention. For example, the device may be a device or chip system of the above-mentioned terminal unit or server. As shown in FIG. 3, the device includes: an acquisition module 201, a first data acquisition module 202, a second data acquisition module 203, a first feature data processing module 204, a second feature data processing module 205, a feature data fusion module 206, and a blood glucose prediction module 207.

The acquisition module 201 is configured to acquire a calibrated blood glucose value to generate first calibrated blood glucose data.

The first data acquisition module 202 is configured to, according to a preset optical signal acquisition duration, a human body optical signal type and three human body optical signal bands, continuously acquire human body optical signals to generate corresponding first, second, and third optical signals, and according to the optical signal acquisition duration, continuously acquire environmental optical signals to generate a fourth optical signal.

The third data acquisition module 203 is configured to, according to the preset metabolic heat signal acquisition duration, continuously acquire human body contact heat signals to generate a first metabolic heat signal, continuously acquire radiation heat signals from a proximal end of a human body to generate a second metabolic heat signal, continuously acquire temperature change information from the proximal end of the human body to form a third metabolic heat signal, continuously acquire humidity change information from the proximal end of the human body to form a fourth metabolic heat signal, continuously acquire calibration output information from a proximal-end radiation sensor used to acquire the radiation heat signals to form a fifth metabolic heat signal, continuously acquire temperature change information from a distal end of the human body to form a sixth metabolic heat signal, and continuously acquire humidity change information from the distal end of the human body to form a seventh metabolic heat signal.

The first feature data processing module 204 is configured to extract and normalize human body optical signal features of the first, second, and third optical signals to generate corresponding first, second, and third optical feature data groups, and extract and normalize environmental optical signal features of the fourth optical signal to generate corresponding fourth optical feature data.

The second feature data processing module 205 is configured to extract and normalize metabolic heat features of the first, second, third, fourth, fifth, sixth, and seventh metabolic heat signals to generate corresponding first, second, third, fourth, fifth, sixth, and seventh metabolic heat feature data.

The feature data fusion module 206 is configured to perform feature fusion on all the obtained feature data to generate a first feature vector.

The blood glucose prediction module 207 is configured to, based on a floating blood glucose prediction model, predict floating blood glucose values for the first feature vector to generate first floating blood glucose data, and generate first predicted blood glucose data according to a sum of the first calibrated blood glucose data and the first floating blood glucose data.

An embodiment of the present invention provides a blood glucose prediction device based on optical signal features and metabolic heat features for executing the steps of the method in the method embodiment described above. The blood glucose prediction method and device based on the same principle of implementation and have similar technical effects, the details of which will not be repeated here.

It should be noted that the individual modules in the above-mentioned device should be understood as being divided by logical function only, and may be wholly or partly integrated into a physical entity, or physically discrete, during actual implementation. Moreover, all the modules may be implemented in a form of software to be called by processing elements, or in a form of hardware, or in such a form that some of the modules are implemented in a form of software to be called by processing elements and some of the modules are implemented in a form of hardware. For example, the acquisition module may be a processing element provided separately, or may be implemented by being integrated into a chip of the above-mentioned device, or, furthermore, may be stored in the memory of the above-mentioned device in a form of program codes to called by a processing element of the above-mentioned device to execute the function of the above determined module. Other modules are implemented in a similar way. Furthermore, these modules may be wholly or partly integrated together, or independently implemented. The processing element described here may be an integrated circuit having a signal processing capacity. During implementation, the individual steps of the above-mentioned method or the above individual modules may be completed by integrated logical circuits in the hardware in processor elements or by instructions in a form of software.

For example, the modules above may be one or more integrated circuits, for example, one or more application specific integrated circuits (ASICs), or one or more digital signal processors (DSPs), or one or more field programmable gate arrays (FPGAs) or the like, which are configured to implement the method above. As another example, when a module above is implemented in a form of program codes to be called by the processing element, the processing element may be a general-purpose processor, for example, a central processing unit (CPU) or other processors capable of calling the program codes. As another example, these modules may be integrated together and then implemented in a form of a system-on-a-chip (SOC).

In the above-mentioned embodiments, the implementation may be performed wholly or partly by software, hardware, firmware, or any of their combinations. When the software is used, the implementation may be performed wholly or partly in a form of a computer program product. The computer program product includes one or more computer instructions. When the computer program instruction is loaded and executed in a computer, the flow processes or functions are produced wholly or partly as described in the embodiments of the invention. The above-mentioned computer may be a general-purpose computer, a special-purpose computer, a computer network, or other programmable devices. The above-mentioned computer instruction may be stored in a computer-readable storage medium, or transmitted from one computer-readable storage medium to another computer-readable storage medium. For example, the above-mentioned computer instruction may be transmitted from one website, computer, server, or data center to another website, computer, server, or data center in a wired (for example, a coaxial cable, an optic fiber, or a digital subscriber line (DSL)) or wireless (for example, infrared, wireless, Bluetooth, microwave or the like). The above-mentioned computer-readable storage medium may be any available medium accessible to a computer, or may be a server, a data server, or other data storage devices with one or more available media integrated. The above-mentioned available medium may be a magnetic medium (for example, a floppy disk, a hard disk or a magnetic tape), an optical medium (for example, DVD), or a semiconductor medium (for example, a solid-state disk (SSD)), or the like.

FIG. 4 is a schematic structural diagram of an electronic apparatus according to a third embodiment of the present invention. The electronic apparatus may be the foregoing terminal unit or server, or may be a terminal unit or server connected to the foregoing terminal unit or server for implementing the method according to the embodiment of the invention. As shown in FIG. 4, the electronic device may include: a processor 301 (for example, CPU), a memory 302, and a transceiver 303. The transceiver 303 is coupled to the processor 301, which controls the transceiving action of the transceiver 303. The memory 302 may store various instructions for completing various processing functions and implementing the method and processing processes provided in the above-mentioned embodiments of the present invention. Preferably, the electronic apparatus involved in the embodiment of the present invention further includes: a power source 304, a system bus 305, and a communication port 306. The system bus 305 is configured to implement communication connections between elements. The above-mentioned communication port 306 is configured for connection and communication between the electronic apparatus and other peripherals.

The system bus mentioned in FIG. 4 may be a peripheral component interconnect (PCI) bus or an extended industry standard architecture (EISA) bus or the like. The system bus may be divided into an address bus, a data bus, a control bus, or the like. For ease of illustration, a bold line is only used in the figure for illustration, but it is not intended to indicate the presence of only one bus or one type of bus. The communication port is configured to implement communication between a database access device and other apparatus (for example, a client, a read-write library, and a read-only library). The memory may include a random-access memory (RAM), or a non-volatile memory (NVM), for example, at least one disk memory.

The above processor may be a general-purpose processor, including a central processing unit (CPU), a network processor (NP), or the like; or a digital signal processor (DSP), an application specific integrated circuit (ASIC), a field programmable gate array (FPGA) or other programmable logic devices, a discrete gate or transistor logic device, or a discrete hardware component.

It should be noted that the embodiment of the invention further provides a computer-readable storage medium storing an instruction. The instruction, when executed in a computer, enables the computer to execute the method and processes provided in the embodiments described above.

The embodiment of the present invention further provides a chip for running an instruction. The chip is configured to execute the method and processes provided in the embodiments described above.

The embodiments of the present invention provide a blood glucose prediction method and device based on optical signal features and metabolic heat features, an electronic apparatus, and a computer-readable storage medium, whereby the human body photoplethysmography signals and environmental optical signals are acquired, the contact heat, radiation heat, temperature, humidity, and radiation sensor calibration signals related to the metabolic heat of the human body are acquired, and the ambient temperature and humidity signals related to the metabolic heat of the human body are acquired; furthermore, the features of the acquired optical and metabolic heat-related signals are extracted and normalized to form a feature vector; and the floating blood glucose prediction model reflecting the correlation among the optical, metabolic heat and blood glucose changes is used to perform prediction for the feature vector to obtain the corresponding floating blood glucose data, such that the predicted blood glucose data can be obtained based on the pre-acquired calibrated blood glucose values plus the floating blood glucose data. The present invention provides, for example, a non-invasive measurement mechanism for long-term blood glucose measurement, which reduces for example inconvenience brought to users and decreases for example the difficulty in measurement, thereby improving the user experience.

Those skilled in the art may be further ware of that the units and algorithm steps in each example described in conjunction with the embodiments disclosed herein may be implemented by electronic hardware, computer software or a combination of the two. For a clear description of the interchangeability between the hardware and the software, the composition and steps of each example have been generally described by function in the description above. Whether these functions are executed in a form of hardware or software depends on the specific application of the technical solution and design constraints. Those skilled in the art may implement the described functions by using different methods for each specific application, but such implementation should not be construed as going beyond the scope of the invention.

The steps of the methods or algorithms described in conjunction with the embodiments disclosed herein may be implemented by using hardware, a software module executed by a processor, or a combination of the two. The software module may be disposed in a random-access memory (RAM), an internal memory, a read-only memory (ROM), an electric programmable ROM, an electric erasable programmable ROM, a register, a hard disk, a removable magnetic disk, a CD-ROM, or any storage medium in an additional form as commonly known in the technical field.

The objects, technical solutions and advantageous effects of the invention are further explained in detail with the specific embodiments described above. It should be understood that the description above only involves the specific embodiments of the invention and is not intended to limit the protection scope of the invention. Any modifications, equivalent substitutions, improvements and the like made within the spirit and principle of the invention shall be construed as being included within the protection scope of the invention.

**Claims**

1. A blood glucose prediction method based on optical signal features and metabolic heat features, comprising:

acquiring a calibrated blood glucose value to generate first calibrated blood glucose data; according to a preset optical signal acquisition duration, a human body optical signal type and three human body optical signal bands, continuously acquiring human body optical signals to generate corresponding first, second, and third optical signals, and according to the optical signal acquisition duration, continuously acquiring environmental optical signals to generate a fourth optical signal; according to a preset metabolic heat signal acquisition duration, continuously acquiring human body contact heat signals to generate a first metabolic heat signal, continuously acquiring radiation heat signals from a proximal end of a human body to generate a second metabolic heat signal, continuously acquiring temperature change information from the proximal end of the human body to form a third metabolic heat signal, continuously acquiring humidity change information from the proximal end of the human body to form a fourth metabolic heat signal, continuously acquiring calibration output information from a proximal-end radiation sensor used to acquire the radiation heat signals to form a fifth metabolic heat signal, continuously acquiring temperature change information from a distal end of the human body to form a sixth metabolic heat signal, and continuously acquiring humidity change information from the distal end of the human body to form a seventh metabolic heat signal; extracting and normalizing human body optical signal features of the first, second, and third optical signals to generate corresponding first, second, and third optical feature data groups;

extracting and normalizing environmental optical signal features of the fourth optical signal to generate corresponding fourth optical feature data;

extracting and normalizing metabolic heat features of the first, second, third, fourth, fifth, sixth, and seventh metabolic heat signals to generate corresponding first, second, third, fourth, fifth, sixth, and seventh metabolic heat feature data;

performing feature fusion on all the obtained feature data to generate a first feature vector; and

based on a floating blood glucose prediction model, predicting floating blood glucose values for the first feature vector to generate first floating blood glucose data; and generating first predicted blood glucose data according to a sum of the first calibrated blood glucose data and the first floating blood glucose data.

2. The blood glucose prediction method based on optical signal features and metabolic heat features according to Claim 1, wherein

the optical signal type comprises a photoplethysmography signal type; and
the three optical signal bands comprise an infrared wave band of 650 nm, a near-infrared wave band of 940 nm, and a near-infrared wave band of 1050 nm.

3. The blood glucose prediction method based on optical signal features and metabolic heat features according to Claim 1, wherein extracting and normalizing the human body optical signal features of the first, second, and third optical signals to generate the corresponding first, second, and third optical feature data groups comprises:

extracting a first specified duration of optical signal segment from the first, second, or third optical signal in an intermediate signal time period to generate a first segment signal;

according to a preset bandpass filtering frequency band, performing bandpass filtering on the first segment signal to generate a corresponding first filtered signal, recognizing peak values of the first filtered signal to obtain a plurality of corresponding first signal peak value data, averaging the plurality of first signal peak value data to generate corresponding first averaged peak value data, flipping the first filtered signal upside down to generate a corresponding first flipped signal, recognizing peak values of the first flipped signal to obtain a plurality of corresponding second signal peak value data, averaging the plurality of second signal peak value data and inverting an averaged result to generate corresponding first averaged valley value data, subtracting the first averaged valley value data from the first averaged peak value data to generate corresponding first feature data, and normalizing the first feature data to generate alternating-current feature data;

according to a preset low-pass filtering frequency threshold, performing low-pass filtering on the first segment signal to generate a corresponding second filtered signal, averaging the second filtered signal to generate corresponding second feature data, and normalizing the second feature data to generate direct-current feature data; and

forming the corresponding first, second, and third optical feature data groups from the obtained alternating-current feature data and direct-current feature data.

4. The blood glucose prediction method based on optical signal features and metabolic heat features according to Claim 1, wherein extracting and normalizing the environmental optical signal features of the fourth optical signal to generate the corresponding fourth optical feature data comprises:

extracting a first specified duration of optical signal segment from the fourth optical signal in an intermediate signal time period to generate a second segment signal;

averaging the second segment signal to generate third feature data; and

normalizing the third feature data to generate the fourth optical feature data.

5. The blood glucose prediction method based on optical signal features and metabolic heat features according to Claim 1, wherein extracting and normalizing metabolic heat features of the first, second, third, fourth, fifth, sixth, and seventh metabolic heat signals to generate corresponding first, second, third, fourth, fifth, sixth, and seventh metabolic heat feature data comprises:

extracting a second specified duration of metabolic heat signal segment from the first, second, third, fourth, fifth, sixth, or seventh metabolic heat signal in a last signal time period to generate a corresponding third segment signal;

averaging the third segment signal to generate corresponding fourth feature data; and normalizing the fourth feature data to generate the corresponding first, second, third, fourth, fifth, sixth, or seventh metabolic heat feature data.

6. The blood glucose prediction method based on optical signal features and metabolic heat features according to Claim 1, wherein

the floating blood glucose prediction model is implemented based on a network structure of a multilayer neural network, and comprises an input layer, a hidden layer, and an output layer, wherein the input layer comprises a first number M of input nodes, the hidden layer comprises a second number N of hidden-layer input nodes and a third number S of hidden-layer output nodes, the output layer comprises an output node, the individual hidden-layer input nodes of the hidden layer are connected to all the input nodes of the input layer, respectively, to form a corresponding first fully connected network, the individual hidden-layer output nodes of the hidden layer are connected to all the hidden-layer input nodes of the hidden layer, respectively, to form a corresponding second fully connected network, the output node of the output layer is connected to all the hidden-layer input nodes, the first number M is associated with a sum of feature classifications of the first feature vector, and the second number N is greater than the third number S which is greater than 0;

during prediction of the floating blood glucose values by the floating blood glucose prediction model, the input layer is called to extract individual feature data of the first feature vector in sequence and then input to the corresponding input nodes to generate corresponding input node data;

the hidden layer is called to perform, based on individual parameter matrices of the first fully connected network, corresponding first fully connected network computation on all the input node data connected to the first fully connected network, and to input computation results to the corresponding hidden-layer input nodes as hidden-layer input node data;

the hidden layer is called to perform, based on individual parameter matrices of the second fully connected network, corresponding second fully connected network computation on all the hidden-layer input node data connected to the second fully connected network, and to input computation results to the corresponding hidden-layer output nodes as hidden-layer output node data; and

the output layer is called to perform, based on a preset activation function, corresponding activation function computation on all the hidden-layer output node data and to output computation results as the first floating blood glucose data.

7. A device for implementing steps of the blood glucose prediction method based on optical signal features and metabolic heat features of any one of Claims 1-6, comprising: an acquisition module, a first data acquisition module, a second data acquisition module, a first feature data processing module, a second feature data processing module, a feature data fusion module, and a blood glucose prediction module;

the acquisition module is configured to acquire a calibrated blood glucose value to generate first calibrated blood glucose data;

the first data acquisition module is configured to, according to a preset optical signal acquisition duration, a human body optical signal type and three human body optical signal bands, continuously acquire human body optical signals to generate corresponding first, second, and third optical signals, and according to the optical signal acquisition duration, continuously acquire environmental optical signals to generate a fourth optical signal;

the second data acquisition module is configured to, according to the preset metabolic heat signal acquisition duration, continuously acquire human body contact heat signals to generate a first metabolic heat signal, continuously acquire radiation heat signals from a proximal end of a human body to generate a second metabolic heat signal, continuously acquire temperature change information from the proximal end of the human body to form a third metabolic heat signal, continuously acquire humidity change information from the proximal end of the human body to form a fourth metabolic heat signal, continuously acquire calibration output information from a proximal-end radiation sensor used to acquire the radiation heat signals to form a fifth metabolic heat signal, continuously acquire temperature change information from a distal end of the human body to form a sixth metabolic heat signal, and continuously acquire humidity change information from the distal end of the human body to form a seventh metabolic heat signal;

the first feature data processing module is configured to extract and normalize human body optical signal features of the first, second, and third optical signals to generate corresponding first, second, and third optical feature data groups, and extract and normalize environmental optical signal features of the fourth optical signal to generate corresponding fourth optical feature data;

the second feature data processing module is configured to extract and normalize metabolic heat features of the first, second, third, fourth, fifth, sixth, and seventh metabolic heat signals to generate corresponding first, second, third, fourth, fifth, sixth, and seventh metabolic heat feature data;

the feature data fusion module is configured to perform feature fusion on all the obtained feature data to generate a first feature vector; and
the blood glucose prediction module is configured to, based on a floating blood glucose prediction model, predict floating blood glucose values for the first feature vector to generate first floating blood glucose data, and generate first predicted blood glucose data according to a sum of the first calibrated blood glucose data and the first floating blood glucose data.

8. An electronic apparatus, comprising: a memory, a processor, and a transceiver, wherein

the processor is configured to be coupled with the memory to read and execute an instruction in the memory for implementing the steps of the method of any one of Claims 1-6; and
the transceiver is coupled with the processor, which controls the transceiver to transceive a message.

9. A computer-readable storage medium storing a computer instruction, wherein the computer instruction, when executed by a computer, enables the computer to execute the instruction for the method of any one of Claims 1-6.

| Acquire a calibrated blood glucose value to generate first calibrated blood glucose data | 1 |

| According to a preset optical signal acquisition duration, a human body optical signal type and three human body optical signal bands, continuously acquire human body optical signals to generate corresponding first, second, and third optical signals, and according to the optical signal acquisition duration, continuously acquire environmental optical signals to generate a fourth optical signal | 2 |

| According to the preset metabolic heat signal acquisition duration, continuously acquire human body contact heat signals to generate a first metabolic heat signal, continuously acquire radiation heat signals from the proximal end of a human body to generate a second metabolic heat signal, continuously acquire temperature change information from the proximal end of the human body to form a third metabolic heat signal, continuously acquire humidity change information from the proximal end of the human body to form a fourth metabolic heat signal, continuously acquire calibration output information from a proximal end radiation sensor used to acquire a radiation heat signal, to form a fifth metabolic heat signal, continuously acquire temperature change information from the distal end of the human body to form a sixth metabolic heat signal, and continuously acquire humidity change information from the distal end of the human body to form a seventh metabolic heat signal | 3 |

| Extract and normalize human body optical signal features of the first, second, and third optical signals to generate corresponding first, second, and third optical feature data groups | 4 |

| Extract and normalize the environmental optical signal features of the fourth optical signal to generate corresponding fourth optical feature data | 5 |

| Extract and normalize the metabolic heat features of the first, second, third, fourth, fifth, sixth, and seventh metabolic heat signals to generate corresponding first, second, third, fourth, fifth, sixth, and seventh metabolic heat feature data | 6 |

| Perform feature fusion on all the obtained feature data to generate a first feature vector | 7 |

| Based on a floating blood glucose prediction model, predict floating blood glucose values for the first feature vector to generate first floating blood glucose data | 8 |

| Acquire first predicted blood glucose data according to the sum of the first calibrated blood glucose data and the first floating blood glucose data | 9 |

FIG. 1

Input layer      Hidden layer      Output layer

First feature vector

$P_1$ indicating an input node of the input layer
$P_2$ indicating a hidden-layer input node of the hidden layer
$P_3$ indicating a hidden-layer output node of the hidden layer
$P_4$ indicating an output node of the output layer

FIG. 2

| Acquisition module | 201 |

| First data acquisition module | 202 |

| Second data acquisition module | 203 |

| First feature data processing module | 204 |

| Second feature data processing module | 205 |

| Feature data fusion module | 206 |

| Blood glucose prediction module | 207 |

FIG. 3

| Processor | 301 |
| Power source | 304 |
| Memory | 302 |

305

| Transceiver | 303 |
| Communication port | 306 |

FIG. 4

## INTERNATIONAL SEARCH REPORT

| International application No. |
| --- |
| **PCT/CN2022/097242** |

### A. CLASSIFICATION OF SUBJECT MATTER

A61B 5/1455(2006.01)i

According to International Patent Classification (IPC) or to both national classification and IPC

### B. FIELDS SEARCHED

Minimum documentation searched (classification system followed by classification symbols)

A61B5/-

Documentation searched other than minimum documentation to the extent that such documents are included in the fields searched

Electronic data base consulted during the international search (name of data base and, where practicable, search terms used)

CNABS, CNTXT, WPI, EPODOC, CNKI: 乐普(北京)医疗器械股份有限公司, 血糖, 光, 热, 温度, 湿度, 模型, blood sugar, light, thermal, temperature, humidity, model

### C. DOCUMENTS CONSIDERED TO BE RELEVANT

| Category* | Citation of document, with indication, where appropriate, of the relevant passages | Relevant to claim No. |
| --- | --- | --- |
| PX | CN 114098724 A (LEPU MEDICAL TECHNOLOGY (BEIJING) CO., LTD.) 01 March 2022 (2022-03-01)<br>claims 1-9 | 1-9 |
| Y | CN 102293654 A (TSINGHUA UNIVERSITY) 28 December 2011 (2011-12-28)<br>description, paragraphs [0021]-[0030], and figures 1-4 | 1-9 |
| Y | CN 104665840 A (GUILIN MAIDISHENG ELECTRONIC TECHNOLOGY CO., LTD.) 03 June 2015 (2015-06-03)<br>description, paragraphs [0032]-[0060], and figures 1-9 | 1-9 |
| A | CN 107802255 A (HANGZHOU DIANZI UNIVERSITY) 16 March 2018 (2018-03-16)<br>entire document | 1-9 |
| A | JP 2019198363 A (CUSTOM K.K.) 21 November 2019 (2019-11-21)<br>entire document | 1-9 |
| A | US 2005043630 A1 (BUCHERT, J. M.) 24 February 2005 (2005-02-24)<br>entire document | 1-9 |
| A | CN 113397538 A (SHENZHEN WAKE UP TECHNOLOGY CO., LTD.) 17 September 2021 (2021-09-17)<br>entire document | 1-9 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **15 August 2022** | **25 August 2022** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2022/097242**

| Patent document cited in search report | | | Publication date (day/month/year) | Patent family member(s) | | | Publication date (day/month/year) |
|---|---|---|---|---|---|---|---|
| CN | 114098724 | A | 01 March 2022 | None | | | |
| CN | 102293654 | A | 28 December 2011 | CN | 102293654 | B | 16 July 2014 |
| CN | 104665840 | A | 03 June 2015 | US | 2016256114 | A1 | 08 September 2016 |
| | | | | CN | 104665840 | B | 21 November 2017 |
| | | | | US | 10695005 | B2 | 30 June 2020 |
| CN | 107802255 | A | 16 March 2018 | CN | 107802255 | B | 29 January 2021 |
| JP | 2019198363 | A | 21 November 2019 | JP | 7061362 | B2 | 28 April 2022 |
| US | 2005043630 | A1 | 24 February 2005 | WO | 2006112837 | A1 | 26 October 2006 |
| CN | 113397538 | A | 17 September 2021 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111386652X